(19) Europäisches Patentamt
European Patent Office
Office européen des brevets

(11) EP 4 321 507 A1

(12) EUROPEAN PATENT APPLICATION
published in accordance with Art. 153(4) EPC

(43) Date of publication:
**14.02.2024 Bulletin 2024/07**

(21) Application number: **22784001.4**

(22) Date of filing: **02.04.2022**

(51) International Patent Classification (IPC):
***C07D 273/02*** (2006.01) ***C07D 277/04*** (2006.01)
***A61K 31/41*** (2006.01) ***A61P 37/00*** (2006.01)
***A61P 29/00*** (2006.01)

(52) Cooperative Patent Classification (CPC):
**A61K 31/41; A61P 29/00; A61P 37/00; C07D 273/02; C07D 277/04**

(86) International application number:
**PCT/CN2022/085113**

(87) International publication number:
**WO 2022/213935 (13.10.2022 Gazette 2022/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.04.2021 CN 202110384689**

(71) Applicants:
- **HELIOEAST PHARMACEUTICAL CO., LTD.**
**JiangXi 330096 (CN)**
- **Helioeast Science & Technology Co., Ltd.**
**JiangXi 330096 (CN)**

(72) Inventors:
- **WU, Lingyun**
**Shanghai 200131 (CN)**
- **YOU, Xu**
**Shanghai 200131 (CN)**
- **ZHAO, Lele**
**Shanghai 200131 (CN)**
- **CHEN, Shuhui**
**Shanghai 200131 (CN)**

(74) Representative: **Maiwald GmbH**
**Elisenhof**
**Elisenstraße 3**
**80335 München (DE)**

(54) **OXADIAZOLE-SUBSTITUTED SPIROCYCLIC COMPOUND AND APPLICATION THEREOF**

(57) Provided is a compound as represented by formula (I) or a pharmaceutically acceptable salt thereof. The compound can be used in the preparation of a drug for treating 1 -sphingosine phosphate receptor 1-related diseases, such as autoimmune diseases or inflammations.

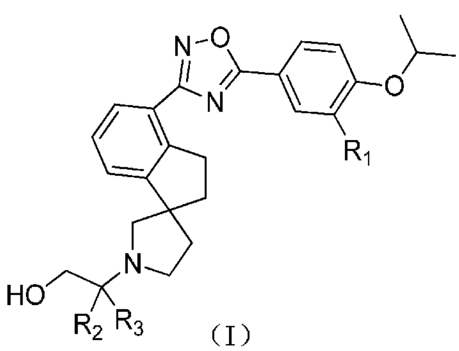
(I)

EP 4 321 507 A1

## Description

CROSS-REFERENCE TO RELATED APPLICATION

The present application claims the priority of the Chinese patent application CN202110384689.2 filed on April 9, 2021, the entirety of which is incorporated herein by reference.

TECHNICAL FIELD

The present disclosure relates to a series of oxadiazole-substituted spirocyclic compounds and a use thereof, specifically to a compound of formula (I) or a pharmaceutically acceptable salt thereof.

BACKGROUND

Sphingosine-1-phosphate (S1P) is a multifunctional lipid mediator with a broad spectrum of physiological activities, including cell proliferation, cell survival, lymphocyte trafficking, cytoskeletal organization, and morphogenesis. Sphingosine is catalyzed by ceramidase and is released from ceramide. In the presence of sphingosine kinase, sphingosine undergoes phosphorylation to produce sphingosine-1-phosphate (S1P), which interacts with sphingosine-1-phosphate receptor (S1PR) to elicit physiological activities.

Sphingosine-1-phosphate receptor 1 (S1PR1), also known as endothelial differentiation gene 1 (EDG1), is a G-protein coupled receptor that belongs to the endothelial differentiation gene (EDG) receptor family. It is a protein encoded by the S1PR1 gene. The sphingosine-1-phosphate receptor (S1PR) comprises five subtypes (S1PR1 to 5), among which sphingosine-1-phosphate receptor 1 (S1PR1) is abundantly distributed on the endothelial cell membrane. Like other G-protein coupled receptors, S1PR1 detects its ligand extracellularly and activates intracellular signaling pathways, leading to a cellular response.

Sphingosine-1-phosphate (S1P) is crucial in the human body, as it primarily regulates the vascular and immune systems. Small molecule S1P1 agonists and inhibitors mimic the binding mechanism of sphingosine-1-phosphate (S1P) to its receptor and have been shown to play significant physiological roles in its signaling system. Activation of sphingosine-1-phosphate receptor 1 (S1PR1) disrupts lymphocyte trafficking, sequestering lymphocytes in lymph nodes and other secondary lymphoid organs, leading to rapid and reversible lymphopenia. Clinical studies have shown that the sequestration of lymphocytes reduces inflammatory or autoimmune responses, which is crucial for immunomodulation.

Currently, the in vivo pharmacodynamic studies of sphingosine-1-phosphate receptor 1 (S1PR1) agonists have been disclosed and intended for the treatment or prevention of autoimmune diseases. The discovery and application of novel sphingosine-1-phosphate receptor 1 (S1PR1) agonists hold great promise.

CONTENT OF THE PRESENT INVENTION

The present disclosure provides a compound of formula (I) or a pharmaceutically acceptable salt thereof,

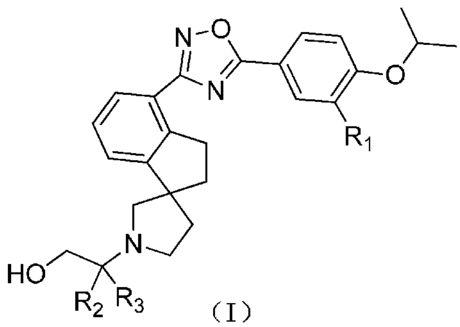

(I)

wherein

$R_1$ is selected from H, F, Cl, Br, I, OH, $NH_2$, CN, $C_{1-3}$ alkyl, and $C_{1-3}$ alkoxy, wherein the $C_{1-3}$ alkyl and $C_{1-3}$ alkoxy are each independently and optionally substituted by 1, 2, or 3 $R_a$;
$R_2$ is selected from H, $C_{1-3}$ alkyl, and $C_{1-3}$ alkoxy, wherein the $C_{1-3}$ alkyl and $C_{1-3}$ alkoxy are each independently and optionally substituted by 1, 2, or 3 $R_b$;
each $R_3$ is independently selected from $C_{1-3}$ alkyl and $C_{1-3}$ alkoxy, wherein the $C_{1-3}$ alkyl and $C_{1-3}$ alkoxy are each

independently and optionally substituted by 1, 2, or 3 $R_c$;
or, $R_2$ and $R_3$ together with the carbon atom to which they are attached form a $C_{3-7}$ cycloalkyl ring;
$R_a$, $R_b$, and $R_c$ are each independently selected from F, Cl, Br, I, OH, $NH_2$, CN, and COOH.

In some embodiments of the present disclosure, the $R_a$, $R_b$, and $R_c$ are each independently selected from F, Cl, and Br, and other variables are as defined in the present disclosure.

In some embodiments of the present disclosure, the $R_1$ is selected from H, F, Cl, Br, CN, $-CH_3$, and

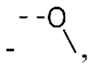,

wherein the $-CH_3$ and $-OCH_3$ are each independently and optionally substituted by 1, 2, or 3 $R_a$, and $R_a$ and other variables are as defined in the present disclosure.

In some embodiments of the present disclosure, each $R_1$ is independently selected from H, F, Cl, Br, CN, $-CH_3$,

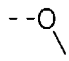, 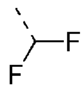, and 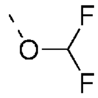,

and other variables are as defined in the present disclosure.

In some embodiments of the present disclosure, the $R_2$ is selected from H, $-CH_3$, - $CH_2CH_3$,

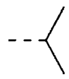, 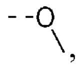, and 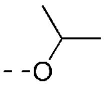,

wherein the $-CH_3$, $-CH_2CH_3$,

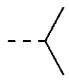, 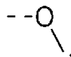, and 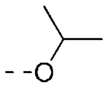

are each independently and optionally substituted by 1, 2, or 3 $R_b$, and $R_b$ and other variables are as defined in the present disclosure.

In some embodiments of the present disclosure, the $R_2$ is selected from H, $-CH_3$, - $CH_2CH_3$,

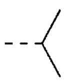, 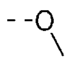, 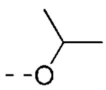, 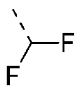, 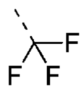, 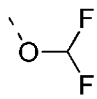, and 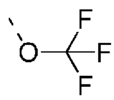,

and other variables are as defined in the present disclosure.

In some embodiments of the present disclosure, the $R_3$ is selected from $-CH_3$, - $CH_2CH_3$,

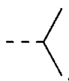, 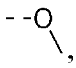, and 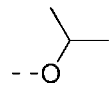,

wherein the $-CH_3$, $-CH_2CH_3$,

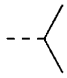, 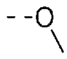, and 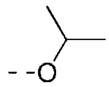

are each independently and optionally substituted by 1, 2, or 3 $R_c$, and $R_c$ and other variables are as defined in the present disclosure.

In some embodiments of the present disclosure, the $R_3$ is selected from $-CH_3$, $-CH_2CH_3$,

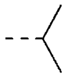, 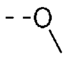, 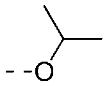, 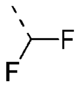, 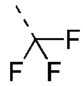, 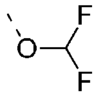, and 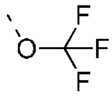,

and other variables are as defined in the present disclosure.

In some embodiments of the present disclosure, the $R_2$ and $R_3$ together with the carbon atom to which they are attached form

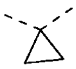, 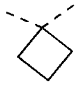, or 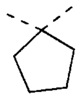,

and other variables are as defined in the present disclosure.

In some embodiments of the present disclosure, the structural moiety

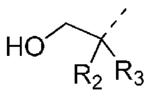

is selected from

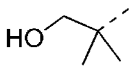, 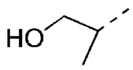, 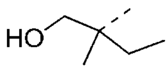, 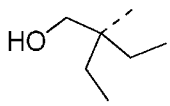, 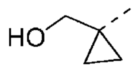, 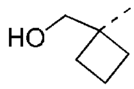,

and

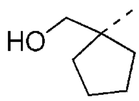,

and other variables are as defined in the present disclosure.

In some embodiments of the present disclosure, the structural moiety

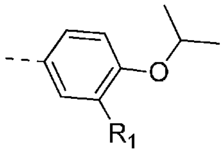

is selected from

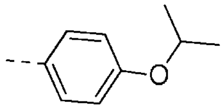, 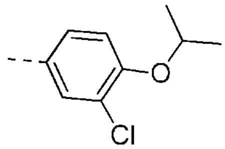, 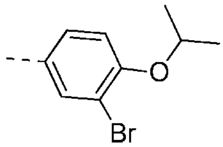,

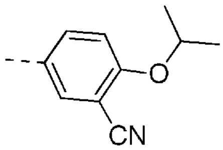, 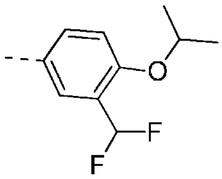, and 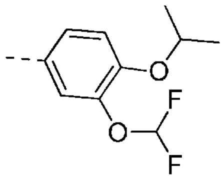,

and other variables are as defined in the present disclosure.

There are still some embodiments of the present disclosure which are obtained by any combination of the above variables.

The present disclosure also provides a compound of the following formulas or a pharmaceutically acceptable salt thereof,

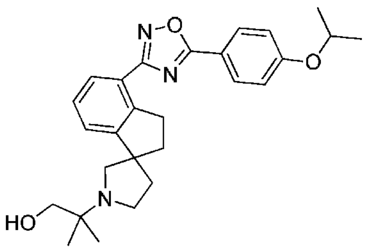, 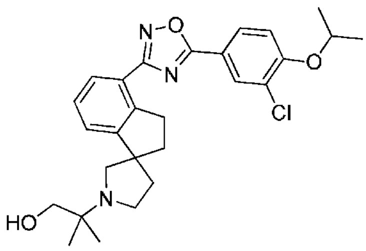, 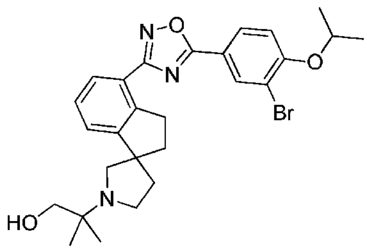,

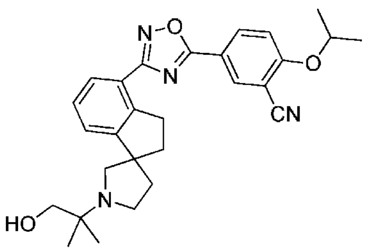, 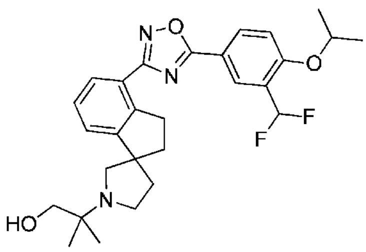, 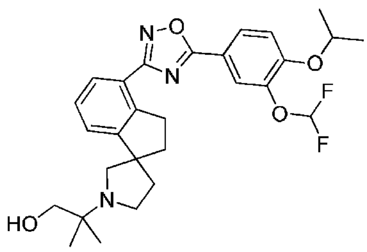,

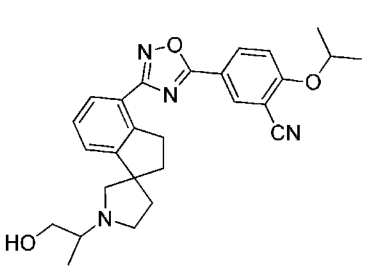, 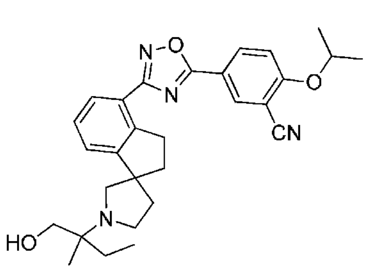, 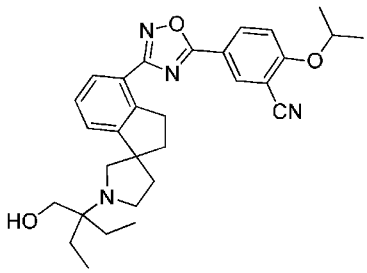,

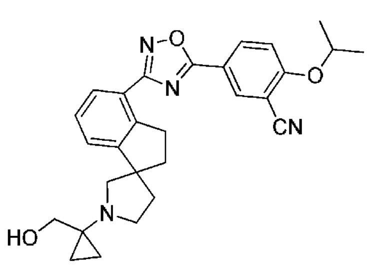, 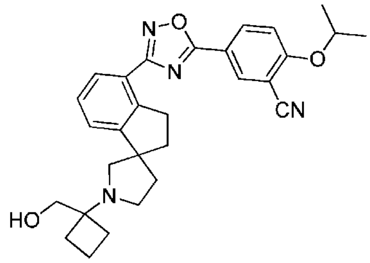, or 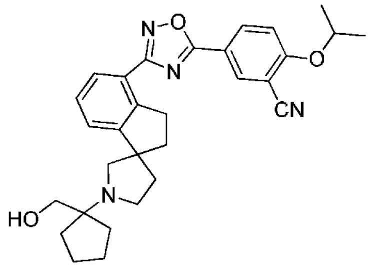.

The present disclosure also provides a compound of the following formulas or a pharmaceutically acceptable salt thereof,

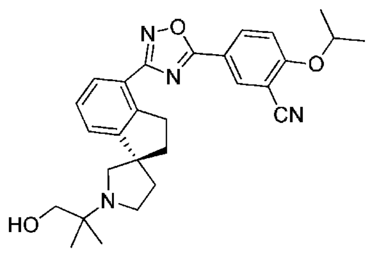, 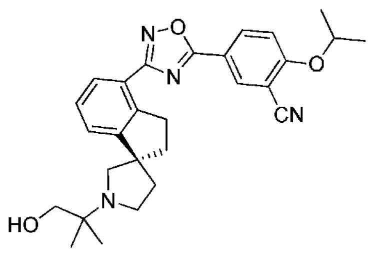, 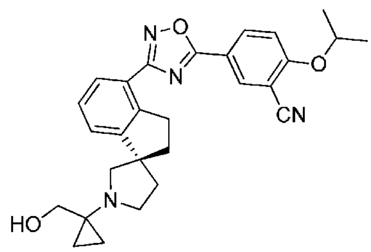,

or

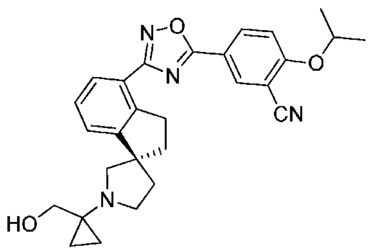.

The present disclosure also provides a compound or a pharmaceutically acceptable salt thereof, wherein the compound is a mixture of

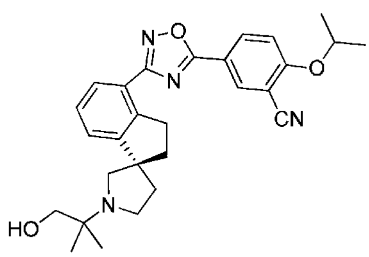

and

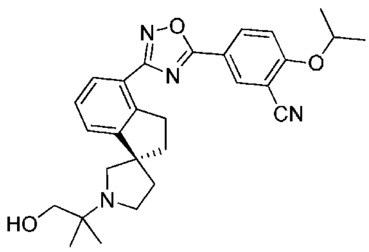

(e.g., a racemic mixture).

The present disclosure also provides a pharmaceutical composition comprising the compound or the pharmaceutically acceptable salt thereof, and at least one pharmaceutically acceptable carrier.

The present disclosure also provides a use of the compound or the pharmaceutically acceptable salt thereof or the pharmaceutical composition in the manufacture of a medicament for treating diseases related to sphingosine-1-phosphate receptor 1.

The present disclosure also provides a use of the compound or the pharmaceutically acceptable salt thereof as a sphingosine-1-phosphate receptor 1 agonist.

In some embodiments of the present disclosure, the diseases are autoimmune diseases or inflammations.

**Technical effect**

The compound of the present disclosure exhibits significant S1PR1 agonist activity and favorable bioavailability.

**Definition and description**

Unless otherwise specified, the following terms and phrases when used herein have the following meanings. A specific term or phrase should not be considered indefinite or unclear in the absence of a particular definition, but should be understood in the ordinary sense. When a trading name appears herein, it is intended to refer to its corresponding commercial product or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by bringing the compound into contact with a sufficient amount of base in a pure solution or a suitable inert solvent. The pharmaceutically acceptable base addition salt includes a salt of sodium, potassium, calcium, ammonium, organic amine or magnesium, or similar salts. When the compound of the present disclosure contains a relatively basic functional group, an acid addition salt can be obtained by bringing the compound into contact with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid, and the like; and salts of amino acid (such as arginine and the like), and a salt of an organic acid such as glucuronic acid and the like. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by a conventional chemical method. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including *cis* and *trans* isomers, (-)- and (+)-enantiomers, (*R*)- and (*S*)-enantiomers, diastereoisomers, (*D*)-isomers, (*Z*)-isomers, and racemic and other mixtures thereof, such as enantiomers or diastereomer enriched mixtures, all of which are within the scope of the present disclosure. Additional asymmetric carbon atoms may be present in substituents such as alkyl. All these isomers and their mixtures are encompassed within the scope of the present disclosure.

Unless otherwise specified, the term "enantiomer" or "optical isomer" refers to stereoisomers that are mirror images of each other.

Unless otherwise specified, the term *"cis-trans* isomer" or "geometric isomer" is caused by the inability to rotate freely of double bonds or single bonds of ring-forming carbon atoms.

Unless otherwise specified, the term "diastereomer" refers to a stereoisomer in which a molecule has two or more chiral centers and the relationship between the molecules is not mirror images.

Unless otherwise specified, "(+)" refers to dextrorotation, "(-)" refers to levorotation, and "(±)" refers to racemic.

Unless otherwise specified, the absolute configuration of a stereogenic center is represented by a wedged solid

bond (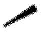) and a wedged dashed bond (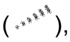), and the relative configuration of a stereogenic center is represented by a straight solid bond (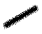) and a straight dashed bond (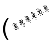), a wave line (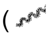) is used to represent a wedged solid bond (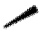) or a wedged dashed bond (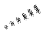), or the wave line (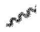) is used to represent a straight solid bond (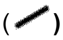) and a straight dashed bond (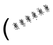). Unless otherwise specified, the term "tautomer" or "tautomeric form" means that at room temperature, the isomers of different functional groups are in dynamic equilibrium and can be transformed into each other quickly. If tautomers possibly exist (such as in solution), the chemical equilibrium of tautomers can be reached. For example, proton tautomer (also called prototropic tautomer) includes interconversion through proton migration, such as keto-enol isomerization and imine-enamine isomerization. Valence tautomer includes some recombination of bonding electrons for mutual transformation. A specific example of keto-enol tautomerization is the tautomerism between two tautomers of pentane-2,4-dione and 4-hydroxypent-3-en-2-one.

Unless otherwise specified, the terms "enriched in one isomer", "enriched in isomers", "enriched in one enantiomer" or "enriched in enantiomers" refer to the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomer excess (ee value) is 80%.

Optically active (*R*)- and (*S*)-isomer, as well as (*D*)- and (*L*)-isomer can be prepared using chiral synthesis or chiral reagents or other conventional techniques. If one kind of enantiomer of certain compound of the present disclosure is to be obtained, the pure desired enantiomer can be obtained by asymmetric synthesis or derivative action of chiral auxiliary followed by separating the resulting diastereomeric mixture and cleaving the auxiliary group. Alternatively, when the molecule contains a basic functional group (such as amino) or an acidic functional group (such as carboxyl), the compound reacts with an appropriate optically active acid or base to form a salt of the diastereoisomer which is then subjected to diastereomeric resolution through the conventional method in the art to give the pure enantiomer. In addition, the enantiomer and the diastereoisomer are generally isolated through chromatography which uses a chiral stationary phase and optionally combines with chemical derivative method (such as carbamate generated from amine).

The compound of the present disclosure may contain an unnatural proportion of atomic isotope at one or more than one atom(s) that constitute the compound. For example, the compound can be radiolabeled with a radioactive isotope, such as tritium ($^3H$), iodine-125 ($^{125}I$), or C-14 ($^{14}C$). For another example, deuterated drugs can be formed by replacing hydrogen with heavy hydrogen, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, etc. All isotopic variations of the compound of the present disclosure, whether radioactive or not, are encompassed within the scope of the present disclosure.

The term "optional" or "optionally" means that the subsequently described event or circumstance may, but does not necessarily, occur, and the description includes instances where the event or circumstance occurs and instances where it does not.

The term "substituted" means one or more than one hydrogen atom(s) on a specific atom are substituted with the substituent, including deuterium and hydrogen variables, as long as the valence of the specific atom is normal and the substituted compound is stable. When the substituent is an oxygen (i.e., =O), it means two hydrogen atoms are substituted. Positions on an aromatic ring cannot be substituted with a ketone. The term "optionally substituted" means an atom can be substituted with a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted by 0 to 2 R, the group can be optionally substituted by up to two R, wherein the definition of R at each occurrence is independent. Moreover, a combination of the substituent and/or the variant thereof is allowed only when the combination results in a stable compound.

When the number of a linking group is 0, such as $-(CRR)_0-$, it means that the linking group is a single bond.

When one of the variables is selected from a single bond, it means that the two groups linked by the single bond are connected directly. For example, when L in A-L-Z represents a single bond, the structure of A-L-Z is actually A-Z.

When a substituent is vacant, it means that the substituent is absent, for example, when X is vacant in A-X,

the structure of A-X is actually A. When the enumerative substituent does not indicate by which atom it is linked to the group to be substituted, such substituent can be bonded by any atom thereof. For example, when pyridyl acts as a substituent, it can be linked to the group to be substituted by any carbon atom on the pyridine ring.

When the enumerative linking group does not indicate the direction for linking, the direction for linking is arbitrary, for example, the linking group L contained in

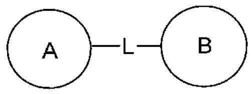

is -M-W-, then -M-W- can link ring A and ring B to form

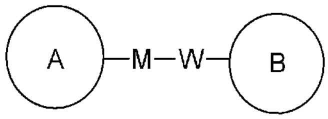

in the direction same as left-to-right reading order, and form

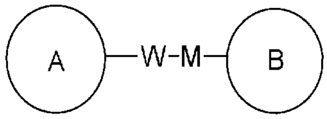

in the direction contrary to left-to-right reading order. A combination of the linking groups, substituents, and/or variables thereof is allowed only when such combination can result in a stable compound.

Unless otherwise specified, when a group has one or more linkable sites, any one or more sites of the group can be linked to other groups through chemical bonds. When the linking site of the chemical bond is not positioned, and there is an H atom at the linkable site, then the number of H atoms at the site will decrease correspondingly with the number of chemical bonds linking thereto so as to meet the corresponding valence. The chemical bond between the site and other groups can be represented by a straight solid bond (/), a straight dashed bond (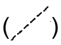) or a wavy line (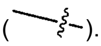). For example, the straight solid bond in $-OCH_3$ means that it is linked to other groups through the oxygen atom in the group; the straight dashed bond in

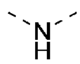

means that it is linked to other groups through the two ends of the nitrogen atom in the group; the wave lines in

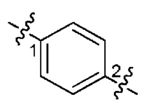

means that the phenyl group is linked to other groups through carbon atoms at position 1 and position 2;

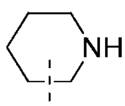

means that it can be linked to other groups through any linkable sites on the piperidinyl by one chemical bond, including at least four types of linkage, including

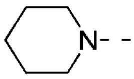, 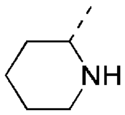, 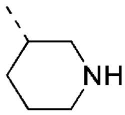,

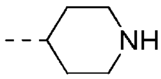.

Even though the H atom is drawn on the -N-,

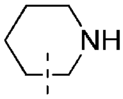

still includes the linkage of

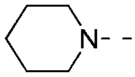,

merely when one chemical bond was connected, the H of this site will be reduced by one to the corresponding monovalent piperidinyl.

Unless otherwise specified, when a double bond structure, such as carbon-carbon double bond, carbon-nitrogen double bond, and nitrogen-nitrogen double bond, exists in the compound, and each of the atoms on the double bond is connected to two different substituents (including the condition where a double bond contains a nitrogen atom, the lone pair of electrons attached on the nitrogen atom is regarded as a substituent connected), if the atom on the double bond in the compound is connected to its substituent by

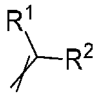,

this refers to the (Z)-isomer, (E)-isomer or a mixture of two isomers of the compound.

Unless otherwise specified, the term "$C_{1-3}$ alkyl" refers to a linear or branched saturated hydrocarbon group consisting of 1 to 3 carbon atoms. The $C_{1-3}$ alkyl includes $C_{1-2}$ and $C_{2-3}$ alkyl, etc.; it can be monovalent (such as methyl), divalent (such as methylene), or multivalent (such as methine). Examples of $C_{1-3}$ alkyl include, but are not limited to, methyl (Me), ethyl (Et), propyl (including n-propyl and isopropyl), etc.

Unless otherwise specified, the term "$C_{1-3}$ alkoxy" refers to an alkyl group consisting of 1 to 3 carbon atoms that are connected to the rest of the molecule through an oxygen atom. The $C_{1-3}$ alkoxy includes $C_{1-2}$, $C_{2-3}$, $C_3$, and $C_2$ alkoxy, etc. Examples of $C_{1-3}$ alkoxy include, but are not limited to, methoxy, ethoxy, propoxy (including n-propoxy and isopropoxy), etc.

Unless otherwise specified, the term "$C_{3-7}$ cycloalkyl" refers to a saturated cyclic hydrocarbon group consisting of 3 to 7 carbon atoms, which includes monocyclic and bicyclic systems, wherein the bicyclic system includes a spiro ring, a fused ring, and a bridged ring. The $C_{3-7}$ cycloalkyl includes $C_{3-6}$, $C_{3-5}$, $C_{4-7}$, $C_{4-6}$, $C_{4-5}$, $C_{5-7}$, or $C_{5-6}$ cycloalkyl, etc.; it can be monovalent, divalent, or multivalent. Examples of $C_{3-7}$ cycloalkyl include, but are not limited to, cyclopropyl, cyclobutyl, cyclopentyl, cyclohexyl, cycloheptyl, norbornyl, spiroheptyl, etc.

The term "leaving group" refers to a functional group or atom which can be replaced by another functional group or atom through a substitution reaction (such as affinity substitution reaction). For example, representative leaving groups include trifluoromethanesulfonate; chlorine, bromine, and iodine; sulfonate group, such as methanesulfonate, toluenesulfonate, p-bromobenzenesulfonate, p-toluenesulfonate, etc; acyloxy, such as acetoxy, trifluoroacetoxy, etc.

The term "protecting group" includes, but is not limited to, "amino protecting group", "hydroxyl protecting group" or "mercapto protecting group". The term "amino protecting group" refers to a protecting group suitable for preventing the side reactions occurring at the nitrogen of an amino. Representative amino protecting groups include, but are not limited to: formyl; acyl, such as alkanoyl (e.g., acetyl, trichloroacetyl, or trifluoroacetyl); alkoxycarbonyl, such as tert-butoxycarbonyl (Boc); arylmethoxycarbonyl, such as benzyloxycarbonyl (Cbz) and 9-fluorenylmethoxycarbonyl (Fmoc);

arylmethyl, such as benzyl (Bn), trityl (Tr), 1,1-bis-(4'-methoxyphenyl)methyl; silyl, such as trimethylsilyl (TMS) and tert-butyldimethylsilyl (TBS), etc. The term "hydroxyl protecting group" refers to a protecting group suitable for preventing the side reactions of hydroxyl. Representative hydroxyl protecting groups include, but are not limited to: alkyl, such as methyl, ethyl, and tert-butyl; acyl, such as alkanoyl (e.g., acetyl); arylmethyl, such as benzyl (Bn), p-methoxybenzyl (PMB), 9-fluorenylmethyl (Fm), and diphenylmethyl (benzhydryl, DPM); silyl, such as trimethylsilyl (TMS) and tert-butyldimethylsilyl (TBS), etc.

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include but are not limited to the examples of the present disclosure.

The structure of the compounds of the present disclosure can be confirmed by conventional methods known to those skilled in the art, and if the disclosure involves an absolute configuration of a compound, then the absolute configuration can be confirmed by means of conventional techniques in the art. For example, in the case of single crystal X-ray diffraction (SXRD), the absolute configuration can be confirmed by collecting diffraction intensity data from the cultured single crystal using a Bruker D8 venture diffractometer with CuK$\alpha$ radiation as the light source and scanning mode: $\varphi/\omega$ scan, and after collecting the relevant data, the crystal structure can be further analyzed by the direct method (Shelxs97).

The compounds of the present disclosure are named according to the conventional naming principles in the art or by ChemDraw® software, and the commercially available compounds use the supplier catalog names.

DETAILED DESCRIPTION OF THE PREFERRED EMBODIMENT

The present disclosure is described in detail by the examples below, but it does not mean that there are any adverse restrictions on the present disclosure. The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include but are not limited to the examples of the present disclosure. For one skilled in the art, it is obvious to make various modifications and improvements to the embodiments of the present disclosure without departing from the spirit and scope of the present disclosure.

**Example 1**

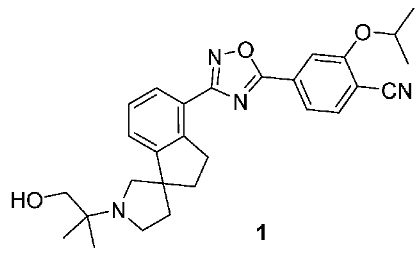

1

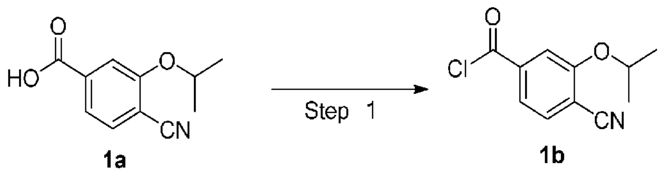


1a 1b

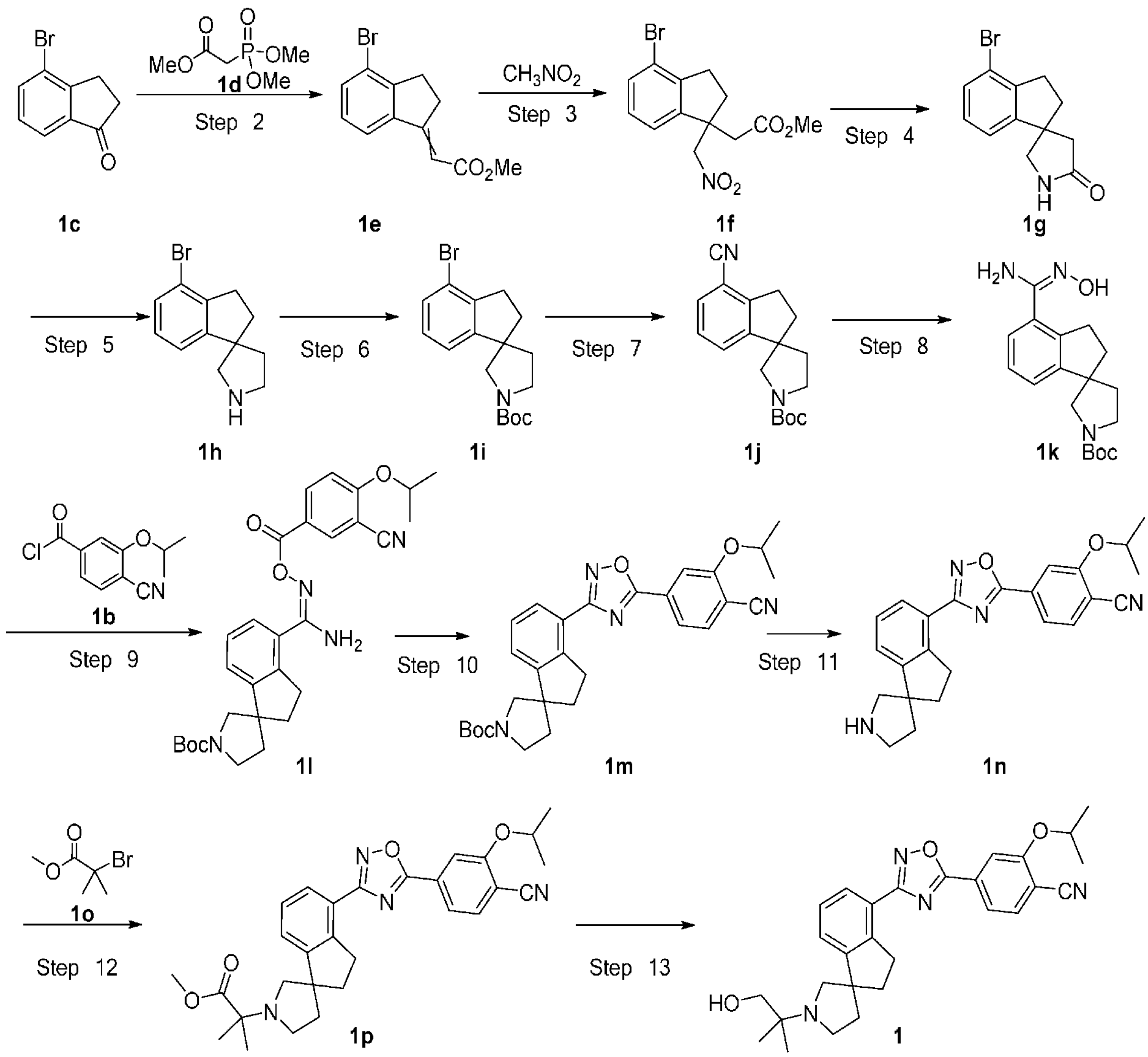


Step 1

Compound **1a** (2 g, 9.75 mmol) was dissolved in dichloromethane (20 mL). To the system was sequentially added oxalyl chloride (3.71 g, 29.24 mmol) and *N,N*-dimethylformamide (71.24 mg, 974.61 μmol) at 0°C. The reaction mixture was stirred at 20°C for 0.5 hours. The reaction mixture was concentrated under reduced pressure to remove the solvent, and the resulting crude product **1b** was directly used in a later step.

Step 2

Compound **1d** (280.43 g, 1.54 mol) was dissolved in *N,N*-dimethylformamide (100 mL). To the system was added potassium tert-butoxide (166.15 g, 1.48 mol) in batches at 25°C. The reaction mixture was stirred at 25°C for 3 hours, then slowly added with compound **1c** (250 g, 1.18 mol) in batches, and stirred at 25°C for 13 hours. Other parallel batches (comprising an addition of 840 g of **1d**) were combined for further processing. To the reaction mixture was added water (1600 mL). The reaction mixture was extracted with ethyl acetate (6 L × 5). The combined organic phases were washed with saturated brine (1.6 L × 1), dried over anhydrous sodium sulfate, filtered, and concentrated to obtain a crude product. The crude product was purified by neutral alumina column chromatography (petroleum ether/ethyl acetate, 2/1 to 1/1, V/V) to obtain compound **1e.** MS-ESI calculated for $[M+H]^+$ 267 and 269, and found 267 and 269.

Step 3

Compound **1e** (767 g, 2.87 mol) was dissolved in dimethyl sulfoxide (3000 mL). To the system was added

cesium carbonate (748.44 g, 2.30 mol) at 25°C. The reaction mixture was heated to 70°C, and to the system was slowly added dropwise nitromethane (525.81 g, 8.61 mol). The reaction mixture was stirred at 70°C for 12 hours. Other parallel batches (comprising an addition of 767 g of **1e**) were combined for further processing. The reaction mixture was added with water (2400 mL), and extracted with ethyl acetate (10 L × 1, 5 L × 1). The combined organic phases were washed with saturated brine (5 L × 2), dried over anhydrous sodium sulfate (2 kg), filtered, and concentrated to obtain a crude product. The crude product was purified by neutral alumina column chromatography (petroleum ether/ethyl acetate, 1/1, V/V) to obtain compound **1f**.

Step 4

Compound **1f** (725 g, 2.21 mol) was dissolved in ethanol (2175 mL) and water (725 mL). To the system was added ammonium chloride (354.54 g, 6.63 mol) at 25°C, and the reaction mixture was heated to 80°C. To the system was then slowly added iron powder (370.14 g, 6.63 mol) in batches, and the reaction mixture was stirred at 80°C for 12 hours. Other parallel batches (comprising an addition of 725 g of **1f**) were combined for further processing. The reaction mixture was filtered, and the filter cake was sequentially rinsed with ethyl acetate (8 L) and water (4 L). The filtrate was concentrated to remove some of the solvent, then added with water (4 L), and extracted with ethyl acetate (4 L × 2). The combined organic phases were washed with saturated brine (3 L × 2), dried over anhydrous sodium sulfate (2 kg), filtered, and concentrated to obtain a crude product. At 25°C, the crude product was slurried with a mixed solvent (*n*-heptane/ethyl acetate, 1/1, V/V), stirred for 12 hours, filtered, and dried to obtain compound **1g.** MS-ESI calculated for $[M+H]^+$ 266 and 268, and found 266 and 268.

Step 5

Compound **1g** (50 g, 178.06 mmol) was dissolved in tetrahydrofuran (350 mL). To the system was added dropwise a solution of borane-dimethylsulfide (10 M, 35.61 mL) at 20°C. The reaction mixture was then heated to 70°C, and stirred at 70°C for 12 hours. Other parallel batches (comprising an addition of 50 g of **1g**) were combined for further processing. The reaction mixture was poured into hydrochloric acid aqueous solution (1 M, 400 mL) at 80°C, and extracted with ethyl acetate (200 mL × 3). The resulting aqueous phase was added with saturated sodium carbonate aqueous solution to adjust the pH to about 9, and extracted with dichloromethane (300 mL × 3). The combined organic phases were dried over anhydrous sodium sulfate (100 g), filtered, and concentrated to obtain a crude product. The crude product **1h** was directly used in the next step without further purification. MS-ESI calculated for $[M+H]^+$ 252 and 254, and found 252 and 254.

Step 6

Compound **1h** (22 g, 87.25 mmol) was dissolved in dichloromethane (130 mL). To the system was added triethylamine (21.19 g, 209.40 mmol) and di-tert-butyl dicarbonate (22.85 g, 104.70 mmol). The reaction mixture was stirred at 20°C for 12 hours. Other parallel batches (comprising an addition of 22 g of **1h**) were combined for further processing. To the system was added water (800 mL). The reaction mixture was extracted with dichloromethane (500 mL × 2). The combined organic phases were dried over anhydrous sodium sulfate (100 g), filtered, and concentrated to obtain a crude product. The crude product was purified by silica gel column chromatography (petroleum ether/ethyl acetate, 100/1 to 10/1, V/V) to obtain compound **1i.** MS-ESI calculated for $[M\text{-}t\text{Bu}+H]^+$ 296 and 298, and found 296 and 298.

Step 7

To *N,N*-dimethylformamide (400 mL) was sequentially added compound **1i** (42.9 g, 121.78 mmol), tris(dibenzylideneacetone) (4.46 g, 4.87 mmol), 2-dicyclohexylphosphino-2',4',6'-triisopropylbiphenyl (4.64 g, 9.74 mmol), and zinc cyanide. The reaction system was replaced with nitrogen, stirred at 90°C for 12 hours, added with water (1000 mL), and filtered. The filter cake was washed with ethyl acetate (200 mL × 2). The aqueous phase was extracted with ethyl acetate (300 mL × 2). The combined organic phases were dried over anhydrous sodium sulfate, filtered, and concentrated to obtain a crude product. The crude product was purified by silica gel column chromatography (petroleum ether/ethyl acetate, 50/1 to 10/1, V/V) to obtain compound **1j**. MS-ESI calculated for $[M\text{-}t\text{Bu}+H]^+$ 243, and found 243.

Step 8

Compound **1j** (12.5 g, 41.89 mmol) was dissolved in ethanol (120 mL). To the system was sequentially added hydroxylamine hydrochloride (4.64 g, 9.74 mmol) and triethylamine (4.64 g, 9.74 mmol). The reaction mixture was stirred at 80°C for 5 hours. The reaction mixture was concentrated, and then water (200 mL) was added thereto. The aqueous

phase was extracted with ethyl acetate (150 mL × 2). The combined organic phases were washed with water (100 mL × 3), dried over anhydrous sodium sulfate, filtered, and concentrated to obtain a crude product. The crude product **1k** was directly used in the next step without further purification. MS-ESI calculated for $[M\text{-}t\text{Bu+H}]^+$ 276, and found 276.

Step 9

Compound **1k** (2.7 g, 7.14 mmol) and compound **1b** (2.08 g, 9.28 mmol) were dissolved in dichloromethane (30 mL). To the system was added dropwise *N,N*-diisopropylethylamine (2.77 g, 21.41 mmol). The reaction mixture was stirred at 20°C for 12 hours. The reaction mixture was concentrated, and then water (80 mL) was added thereto. The aqueous phase was extracted with dichloromethane (40 mL × 2). The combined organic phases were dried over anhydrous sodium sulfate (10 g), filtered, and concentrated to obtain a crude product. The crude product **1l** was directly used in the next step without further purification. MS-ESI calculated for $[M\text{-}t\text{Bu+H}]^+$ 463, and found 463.

Step 10

Compound **1l** (5 g, 9.64 mmol) was dissolved in acetonitrile (50 mL). To the system was added sodium hydroxide (1.54 g, 38.57 mmol). The reaction mixture was stirred at 27°C for 12 hours. To the system was added water (80 mL). The aqueous phase was extracted with ethyl acetate (40 mL × 2). The combined organic phases were dried over anhydrous sodium sulfate (5 g), filtered, and concentrated to obtain a crude product. The crude product was purified by silica gel column chromatography (petroleum ether/ethyl acetate, 3/1, V/V) to obtain compound **1m.** MS-ESI calculated for $[M\text{-}t\text{Bu+H}]^+$ 445, and found 445.

Step 11

Compound **1m** (2.1 g, 4.20 mmol) was dissolved in ethyl acetate (20 mL). To the system was added a solution of hydrogen chloride in ethyl acetate (4 M, 20 mL). The reaction mixture was stirred at 20°C for 0.5 hours. The reaction mixture was concentrated under reduced pressure to obtain a crude product. The crude product, hydrochloride of compound **1n**, was directly used in the next step without further purification. MS-ESI calculated for $[M+H]^+$ 401, and found 401.

Step 12

The hydrochloride of compound **1n** (1 g, 2.29 mmol) was dissolved in acetonitrile (15 mL). To the system was sequentially added potassium iodide (189.96 mg, 1.14 mmol), potassium carbonate (948.93 mg, 6.87 mmol), and compound **1o** (621.47 mg, 3.43 mmol). The reaction mixture was stirred at 85°C for 12 hours. To the system was added water (30 mL). The aqueous phase was extracted with ethyl acetate (20 mL × 2). The combined organic phases were dried over anhydrous sodium sulfate (5 g), filtered, and concentrated to obtain a crude product. The crude product was purified by silica gel column chromatography (petroleum ether/ethyl acetate, 5/1 to 3/1, V/V) to obtain compound **1p**. MS-ESI calculated for $[M+H]^+$ 501, and found 501.

Step 13

Compound **1p** (810 mg, 1.62 mmol) was dissolved in tetrahydrofuran (10 mL). To the system was added lithium borohydride (110 mg, 5.05 mmol). The reaction mixture was stirred at 20°C for 0.5 hours, then heated to 70°C, and stirred continuously for 12 hours. To the system was added hydrochloric acid aqueous solution (1 N, 30 mL). The aqueous phase was extracted with ethyl acetate (30 mL × 2). The combined organic phases were dried over anhydrous sodium sulfate (10 g), filtered, and concentrated to obtain a crude product. The crude product was purified by preparative HPLC (chromatographic column: Waters Xbridge C18 150 × 50 mm × 10 μm; mobile phase: 10 mmol/L ammonium bicarbonate aqueous solution-acetonitrile; gradient: acetonitrile from 62% to 92%, 10 minutes) to obtain compound **1.** MS-ESI calculated for $[M+H]^+$ 473, and found 473. $^1$H NMR (400 MHz, $CD_3OD$) δ = 8.47 - 8.41 (m, 2H), 8.01 (d, *J* = 7.6 Hz, 1H), 7.54 (d, *J* = 7.4 Hz, 1H), 7.48 - 7.37 (m, 2H), 5.00 - 4.94 (m, 1H), 3.47 (s, 2H), 3.31 - 3.27 (m, 2H), 3.11 - 3.03 (m, 1H), 3.03 - 2.95 (m, 3H), 2.30 - 2.22 (m, 1H), 2.21 - 2.13 (m, 1H), 2.11 - 1.96 (m, 2H), 1.48 (d, *J* = 6.1 Hz, 6H), 1.14 (s, 6H).

**Bioactivity evaluations**

**Test example 1: Evaluating the in vitro agonist activity of the compound of the present disclosure on S1PR1**

Experimental purpose: To assess the agonist activity of the compound on S1PR1

I. Cell treatment

1. The PathHunter cell line was thawed according to standard procedures.
2. The cells were seeded in a 384-well plate with a volume of 20 μL, and incubated at 37°C for an appropriate duration.

II. Agonist

1. For the agonist evaluation, the cells were co-cultured with the test samples to induce a reaction.
2. The test stock solution was diluted 5-fold using the buffer.
3. 5 μL of the 5-fold diluted solution was added to the cells, and incubated at 37°C for 90 to 180 minutes. The vehicle concentration was maintained at 1%.

III. Signal detection

1. Either 12.5 μL or 15 μL of the PathHunter detection reagent (at a 50% v/v ratio) was added each time, followed by a 1-hour incubation at room temperature to allow for detection signal generation.
2. Chemiluminescence signal detection was conducted using a PerkinElmer Envision™ microplate reader.

IV. Data analysis

1. The activity of the compound was analyzed using the CBIS suite for data analysis (ChemInnovation, CA).
2. Formula for calculation:

% Activity = 100% × (Average sample RLU - Average vehicle RLU) / (Average maximum control ligand RLU - Average vehicle RLU)

The results of the experiment are shown in Table 1:

**Table 1. Results of the S1PR1 agonist activity test**

| Test Sample | S1PR1 Agonist Activity, Emax |
|---|---|
| Compound 1 | 0.657 nM, 110% |

Conclusion: The compound of the present disclosure demonstrates significant and even unexpected S1PR1 agonist activity.

**Test example 2: Pharmacokinetic study on the compound of the present disclosure**

Experimental purpose: To investigate the pharmacokinetics of the compound in SD rats.

Experimental materials:

Sprague Dawley rats (male, 200 to 300 g, 7 to 9 weeks old, sourced from Shanghai Slac)

Experimental procedures:

The pharmacokinetic characteristics of the compound in rodents after intravenous injection and oral administration were evaluated in line with a standard protocol. The rats were given a single intravenous injection and oral administration. For oral administration, the vehicle employed was an aqueous solution of 0.5% methylcellulose and 0.2% Tween 80. For intravenous injection, the vehicle employed was an aqueous solution of DMSO and 10% hydroxypropyl-β-cyclodextrin in a ratio of 5:95. Whole blood samples were collected over a 48-hour period. The samples were then centrifuged at 3000 g for 15 minutes, through which the supernatant (i.e. the plasma sample) was obtained. Four times the volume of an acetonitrile solution containing an internal standard was added to the plasma samples to precipitate proteins. After centrifugation, the supernatant was collected, added with an equal volume of water, and centrifuged again. The final supernatant was then used as the test sample. Blood concentrations of the compound were quantitatively analyzed using the LC-MS/MS method. Pharmacokinetic parameters, such as peak concentration, time to peak concentration, clearance rate, half-life, area under the curve (AUC), and bioavailability, were then calculated.

The results of the experiment are shown in Table 2:

**Table 2. Results of the pharmacokinetic testing**

| **Test Sample** | Peak Concentration **Cmax (nmol/L)** | Clearance Rate CL **(mL/min/k g)** | Tissue Distribution **$V_{dss}$ (L/kg)** | Half-life **$T_{1/2}$ (PO, h)** | Area Under the Curve **$AUC_{0\text{-}last}$ PO (nM.hr)** | Bioavailability F **(%)** |
|---|---|---|---|---|---|---|
| Compound 1 Intravenous 1 mg/kg Oral 2 mg/kg | 123 | 16.4 | 19.2 | 13.1 | 3041 | 75.7 |

Conclusion: The compound of the present disclosure demonstrates favorable bioavailability, relatively high AUC, and relatively extended half-life in the pharmacokinetics of SD rats.

## Claims

1. A compound of formula (I) or a pharmaceutically acceptable salt thereof,

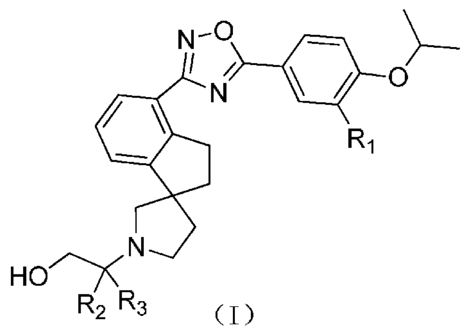

(I)

,

wherein

$R_1$ is selected from H, F, Cl, Br, I, OH, $NH_2$, CN, $C_{1-3}$ alkyl, and $C_{1-3}$ alkoxy, wherein the $C_{1-3}$ alkyl and $C_{1-3}$ alkoxy are each independently and optionally substituted by 1, 2, or 3 $R_a$;
$R_2$ is selected from H, $C_{1-3}$ alkyl, and $C_{1-3}$ alkoxy, wherein the $C_{1-3}$ alkyl and $C_{1-3}$ alkoxy are each independently and optionally substituted by 1, 2, or 3 $R_b$;
$R_3$ is selected from $C_{1-3}$ alkyl and $C_{1-3}$ alkoxy, wherein the $C_{1-3}$ alkyl and $C_{1-3}$ alkoxy are each independently and optionally substituted by 1, 2, or 3 $R_c$;
or, $R_2$ and $R_3$ together with the carbon atom to which they are attached form a $C_{3-7}$ cycloalkyl ring;
$R_a$, $R_b$, and $R_c$ are each independently selected from F, Cl, Br, I, OH, $NH_2$, CN, and COOH.

2. The compound or the pharmaceutically acceptable salt thereof according to claim 1, wherein $R_a$, $R_b$, and $R_c$ are each independently selected from F, Cl, and Br.

**3.** The compound or the pharmaceutically acceptable salt thereof according to claim 1 or 2, wherein $R_1$ is selected from H, F, Cl, Br, CN, $-CH_3$, and

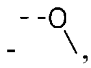,

wherein the $-CH_3$ and

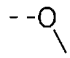

are each independently and optionally substituted by 1, 2, or 3 $R_a$.

**4.** The compound or the pharmaceutically acceptable salt thereof according to claim 3, wherein each $R_1$ is independently selected from H, F, Cl, Br, CN, $-CH_3$,

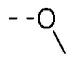, 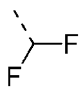,

and

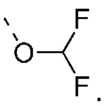.

**5.** The compound or the pharmaceutically acceptable salt thereof according to claim 1 or 2, wherein $R_2$ is selected from H, $-CH_3$, $-CH_2CH_3$,

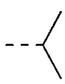, 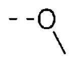, and 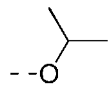,

and the $-CH_3$, $-CH_2CH_3$,

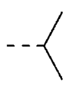, 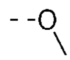, and 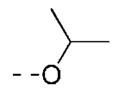

are each independently and optionally substituted by 1, 2, or 3 $R_b$.

**6.** The compound or the pharmaceutically acceptable salt thereof according to claim 5, wherein $R_2$ is selected from H, $-CH_3$, $-CH_2CH_3$,

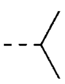, 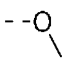, 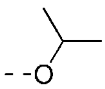, 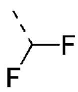, 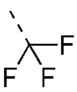,

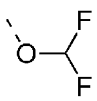, and 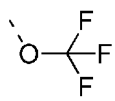.

**7.** The compound or the pharmaceutically acceptable salt thereof according to claim 1 or 2, wherein $R_3$ is selected from $-CH_3$, $-CH_2CH_3$,

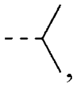, 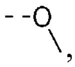, and 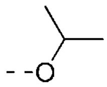,

wherein the $-CH_3$, $-CH_2CH_3$,

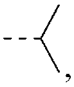, 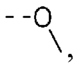, and 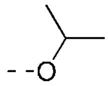

are each independently and optionally selected from 1, 2, or 3 $R_c$.

**8.** The compound or the pharmaceutically acceptable salt thereof according to claim 7, wherein $R_3$ is selected from $-CH_3$, $-CH_2CH_3$,

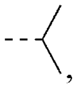, 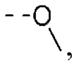, 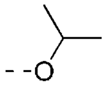, 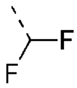, 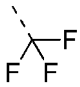, 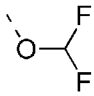,

and

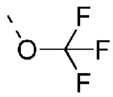.

**9.** The compound or the pharmaceutically acceptable salt thereof according to claim 1, wherein $R_2$ and $R_3$ together with the carbon atom to which they are attached form

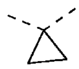,

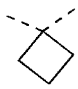, or 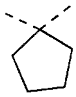.

**10.** The compound or the pharmaceutically acceptable salt thereof according to any one of claims 6, 8, or 9, wherein the structural moiety

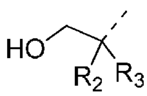

is selected from

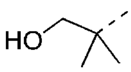,

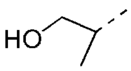, 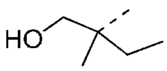, 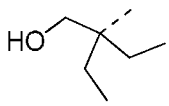, 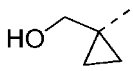, 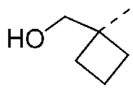, and 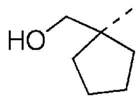.

**11.** The compound or the pharmaceutically acceptable salt thereof according to claim 1 or 2, wherein the structural moiety

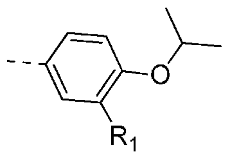

is selected from

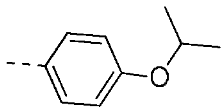,

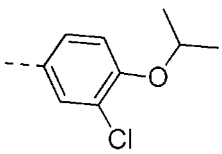, 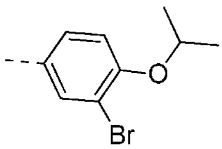, 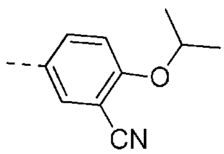, 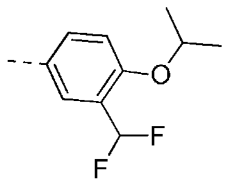,

and

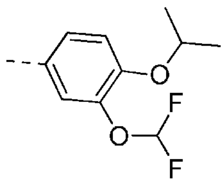.

**12.** A compound or a pharmaceutically acceptable salt thereof, wherein the compound is

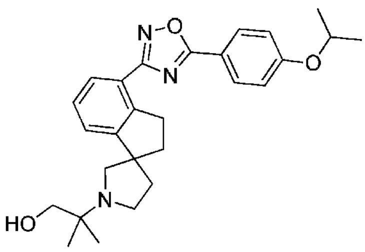, 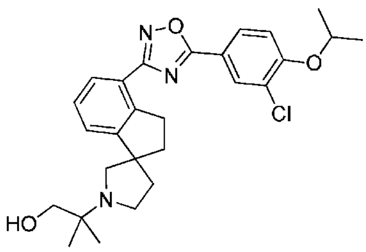, 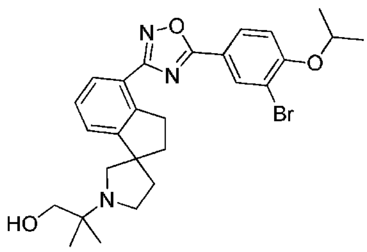,

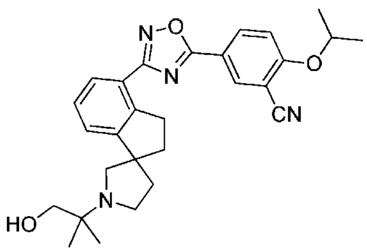, 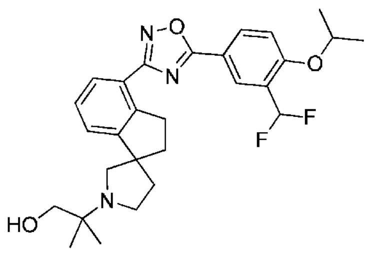, 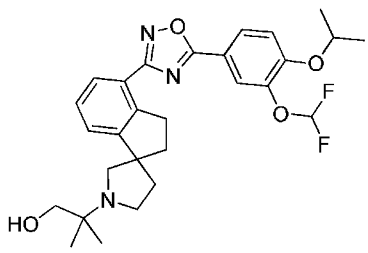,

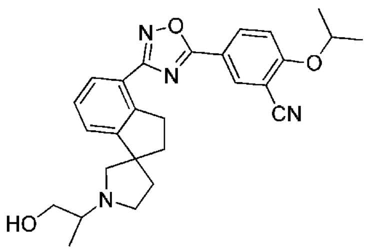, 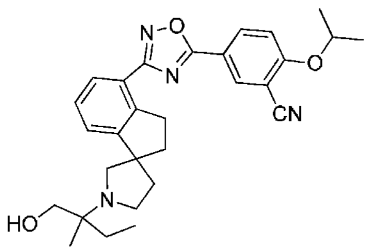, 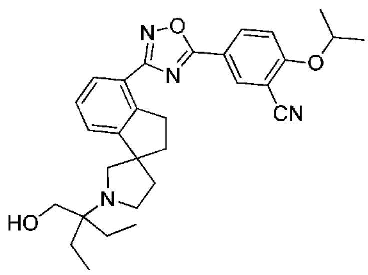,

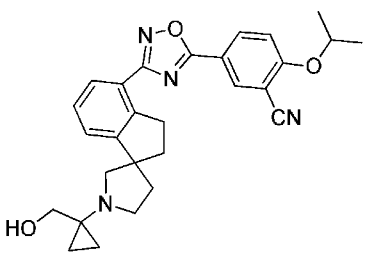, 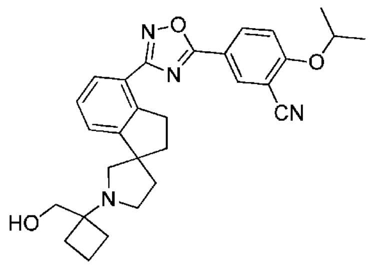, or 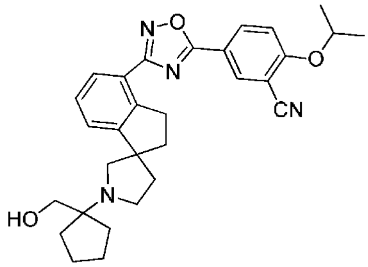.

**13.** The compound or the pharmaceutically acceptable salt thereof according to claim 12, wherein the compound is

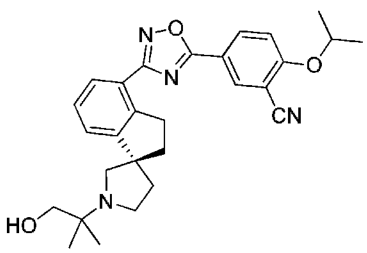, 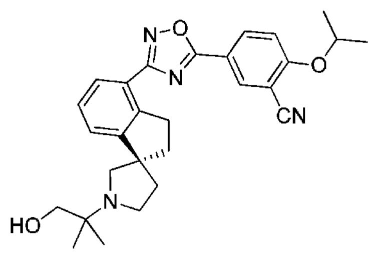, 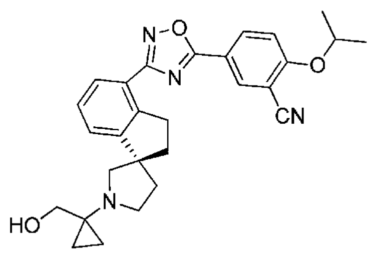,

or

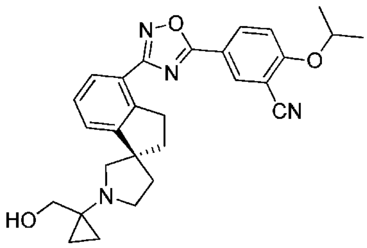.

**14.** A pharmaceutical composition comprising the compound or the pharmaceutically acceptable salt thereof according to any one of claims 1 to 13, and at least one pharmaceutically acceptable carrier.

**15.** A use of the compound or the pharmaceutically acceptable salt thereof according to any one of claims 1 to 13, or

the pharmaceutical composition according to claim 14 as a sphingosine-1-phosphate receptor 1 agonist or in the manufacture of a medicament for treating diseases related to sphingosine-1-phosphate receptor 1.

**16.** The use according to claim 15, wherein the diseases are autoimmune diseases or inflammations.

**INTERNATIONAL SEARCH REPORT**

International application No.
**PCT/CN2022/085113**

**A. CLASSIFICATION OF SUBJECT MATTER**

C07D 273/02(2006.01)i；C07D 277/04(2006.01)i；A61K 31/41(2006.01)i；A61P 37/00(2006.01)i；A61P 29/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNPAT, CNKI, EPODOC, WPI, 百度学术, BAIDU SCHOLAR, 谷歌学术, GOOGLE SCHOLAR, REGISTRY (STN), CAPLUS (STN): 南昌弘益, 1-磷酸鞘氨醇受体, S1PR1, 自免疫, 炎症, structural formula search

**C. DOCUMENTS CONSIDERED TO BE RELEVANT**

| Category* | Citation of document, with indication, where appropriate, of the relevant passages | Relevant to claim No. |
|---|---|---|
| PX | WO 2021175223 A1 (MEDSHINE DISCOVERY INC.) 10 September 2021 (2021-09-10) description, pages 2-4, embodiments, and tables 1-5, and claims 1 and 17 | 1-16 |
| X | WO 2018157813 A1 (MEDSHINE DISCOVERY INC.) 07 September 2018 (2018-09-07) embodiments, and tables 1-2, and claims 1-2, 7 and 18-21 | 1-16 |
| A | WO 2012158550 A2 (RECEPTOS INC. et al.) 22 November 2012 (2012-11-22) entire document | 1-16 |
| A | CN 102471328 A (ACTELION PHARMACEUTICALS LTD.) 23 May 2012 (2012-05-23) entire document | 1-16 |
| A | CN 102762100 A (RECEPTOS, INC.) 31 October 2012 (2012-10-31) entire document | 1-16 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 June 2022** | **30 June 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**
**Information on patent family members**

International application No.
**PCT/CN2022/085113**

| Patent document cited in search report | Publication date (day/month/year) | Patent family member(s) | Publication date (day/month/year) |
|---|---|---|---|
| WO 2021175223 A1 | 10 September 2021 | None | |
| WO 2018157813 A1 | 07 September 2018 | EP 3590929 A1 | 08 January 2020 |
| | | CN 110325517 A | 11 October 2019 |
| | | US 2020048235 A1 | 13 February 2020 |
| | | JP 2020509013 A | 26 March 2020 |
| WO 2012158550 A2 | 22 November 2012 | JP 2014517836 A | 24 July 2014 |
| | | US 2015299179 A1 | 22 October 2015 |
| | | ES 2758841 T3 | 06 May 2020 |
| | | EP 2706999 A2 | 19 March 2014 |
| CN 102471328 A | 23 May 2012 | US 2012108638 A1 | 03 May 2012 |
| | | ZA 201201125 B | 30 July 2014 |
| | | CA 2767585 A1 | 20 January 2011 |
| | | RU 2012105134 A | 27 August 2013 |
| | | HR P20140098 T1 | 14 March 2014 |
| | | CY 1114759 T1 | 14 December 2016 |
| | | TW 201107313 A | 01 March 2011 |
| | | SG 178042 A1 | 29 March 2012 |
| | | BR 112012000763 A2 | 13 March 2018 |
| | | ES 2441845 T3 | 06 February 2014 |
| | | JP 5036923 B1 | 26 September 2012 |
| | | SI 2454255 T1 | 31 January 2014 |
| | | NZ 598173 A | 31 January 2014 |
| | | HK 1170479 A1 | 01 March 2013 |
| | | MX 2012000414 A | 08 February 2012 |
| | | MY 153617 A | 27 February 2015 |
| | | EP 2454255 A1 | 23 May 2012 |
| | | PL 2454255 T3 | 30 April 2014 |
| | | DK 2454255 T3 | 16 December 2013 |
| | | IL 217490 D0 | 29 February 2012 |
| | | KR 20120069662 A | 28 June 2012 |
| | | AU 2010272219 A1 | 08 March 2012 |
| | | WO 2011007324 A1 | 20 January 2011 |
| | | AR 077413 A1 | 24 August 2011 |
| | | PT 2454255 E | 14 January 2014 |
| | | MA 33528 B1 | 01 August 2012 |
| CN 102762100 A | 31 October 2012 | NZ 599915 A | 25 July 2014 |
| | | WO 2011060392 A1 | 19 May 2011 |
| | | CN 105061350 A | 18 November 2015 |
| | | HR P20210446 T1 | 14 May 2021 |
| | | IL 219691 D0 | 31 July 2012 |
| | | EA 201690391 A1 | 31 August 2016 |
| | | LT 3406142 T | 10 June 2021 |
| | | CA 2986521 A1 | 19 May 2011 |
| | | NO 2020039 I1 | 18 November 2020 |
| | | EP 2498610 A1 | 19 September 2012 |
| | | NO 2498610 T3 | 11 August 2018 |
| | | HR P20180874 T1 | 13 July 2018 |
| | | KR 20160149317 A | 27 December 2016 |
| | | TR 201807912 T4 | 21 June 2018 |
| | | HU S2000045 I1 | 28 December 2020 |

Form PCT/ISA/210 (patent family annex) (January 2015)

**INTERNATIONAL SEARCH REPORT**
**Information on patent family members**

International application No.
**PCT/CN2022/085113**

| Patent document cited in search report | Publication date (day/month/year) | Patent family member(s) | | | Publication date (day/month/year) |
|---|---|---|---|---|---|
| | | ES | 2673160 | T3 | 20 June 2018 |
| | | RS | 57253 | B1 | 31 August 2018 |
| | | US | 2017320839 | A1 | 09 November 2017 |
| | | MY | 161854 | A | 15 May 2017 |
| | | RS | 61829 | B1 | 30 June 2021 |
| | | HU | E037535 | T2 | 28 September 2018 |
| | | EP | 3868377 | A1 | 25 August 2021 |
| | | BR | 112012011427 | A2 | 06 October 2015 |
| | | EP | 3406142 | A1 | 28 November 2018 |
| | | ES | 2858337 | T3 | 30 September 2021 |
| | | US | 2015299149 | A1 | 22 October 2015 |
| | | SG | 10201407357 P | A | 30 December 2014 |
| | | CY | 1120338 | T1 | 10 July 2019 |
| | | CA | 2780772 | A1 | 19 May 2011 |
| | | EA | 201290323 | A1 | 30 May 2013 |
| | | IL | 267956 | D0 | 26 September 2019 |
| | | LT | 2498610 | T | 11 June 2018 |
| | | PT | 2498610 | T | 19 June 2018 |
| | | HK | 1213874 | A1 | 15 July 2016 |
| | | JP | 2014122208 | A | 03 July 2014 |
| | | SI | 2498610 | T1 | 31 July 2018 |
| | | HU | E054000 | T2 | 30 August 2021 |
| | | SI | 3406142 | T1 | 31 August 2021 |
| | | PL | 3406142 | T3 | 30 August 2021 |
| | | BR | 122018077504 | B1 | 11 May 2021 |
| | | DK | 3406142 | T3 | 22 March 2021 |
| | | JP | 2015038145 | A | 26 February 2015 |
| | | US | 2011172202 | A1 | 14 July 2011 |
| | | PH | 12015502708 | A1 | 17 October 2016 |
| | | PT | 3406142 | T | 26 March 2021 |
| | | LT | PA2020533 | I1 | 10 December 2020 |
| | | JP | 2013510885 | A | 28 March 2013 |
| | | KR | 20120102704 | A | 18 September 2012 |
| | | AU | 2010319983 | A1 | 31 May 2012 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202110384689